# EUROPEAN PATENT APPLICATION

(11) **EP 3 881 901 A1**
(43) Date of publication of application: **22.09.2021**
(21) Application number: 20164065.3
(22) Date of filing: 18.03.2020
(51) Int. Cl.: A61Q 17/00, A61K 8/22, A61K 8/90

(54) **IMPROVED LIQUID COMPOSITION FOR CLEANING, SANITIZING AND/OR DISINFECTING**

(71) Applicant: Corticalis AS, 0349 Oslo (NO)
(72) Inventor: LYNGSTADAAS, Ståle Petter, 1450 Nesoddtangen (NO); HAUGEN, Håvard J., 0376 Oslo (NO)
(74) Representative: Aera A/S

(57) **Abstract**

The present invention relates to an improved antimicrobial and/or anti-inflammatory composition comprising H₂O₂ at a final concentration of between 0.1 - 7%v/v, and a composite hydrogel formulation comprising pluronic acid at a concentration of 0.1-10%w/v. The composition disclosed herein is antimicrobial and/or anti-inflammatory and is particular useful in cleaning, sterilizing, sanitizing and/or debriding biological surfaces and surface of biomaterials *in situ*, such as a hand sanitizer and/or for wound care and/or chronic ulcer care.

## Description

### TECHNICAL FIELD

The present invention relates to a new multifunctional debridement and/or antifouling composition comprising H₂O₂ at a final concentration of between 0.1 - 5%v/v, and a composite hydrogel formulation comprising pluronic acid at a concentration of 10 - 40%w/v, wherein the composition is in liquid form at room temperature, such as at a temperature of at the most 37°C. The composition disclosed herein is antimicrobial and/or anti-inflammatory and is particular useful in periimplantitis treatment and implant health maintenance, in periodontitis and periodontal health and in wound care and chronic ulcer care.

The present invention further relates to the use of a composition according to the present invention for cleaning and/or debriding a biological surface and/or a biomaterial surface, such as in particular an implant *in situ* and/or a surface in the oral cavity. The composition according to the present invention can be used together with an implant cleaning and/or debridement tool, e.g. a brush, burr or a curette.

In one embodiment, the composition according to the present invention is provided in a kit, wherein the at least two components, H₂O₂ and pluronic acid, are optionally kept separate and mixed instantly at the concomitant and/or simultaneous application.

### BACKGROUND

Biological surface and/or biomaterial surfaces *in situ* are prone to fouling, i.e. the build-up of biofilms and necrotic tissue, as they are in constant contact with and frequently colonized by a plethora of microorganisms. They therefore require periodical cleaning and/or debridement with compositions that are antibacterial and anti-inflammatory without causing harm to the surrounding biological tissue in the patient and without causing microbial resistance.

In particular, the skin of the body, such as on the hands, the feet or the face is often exposed to microorganisms and needs to be cleaned, sterilized and/or sanitized regularly to inhibit settlement and/or transfer of pathogens and/or microbes.

### Biofilms

Biofilms are structured communities of microorganisms that can be firmly attached to a surface and enmeshed in a self-produced three-dimensional (3D) extracellular matrix. Biofilms can form on, or in, living or non-living surfaces and can exist in natural and industrial settings.

Biofilms can be formed on the surface or within implanted medical tubing and medical devices, as well as on the surface and within the human body, e.g. on mucosal surfaces, or on surfaces of other bodily orifices, or in open wounds, which can lead to infections in patients. The inflammatory responses to this in turn leads to the build-up of necrotic tissue, which in turn leads to follow-up inflammatory responses of the body. In particular, biofilms can develop within the oral cavity and often results in oral diseases such as dental caries or periodontitis, gingivitis or peri-implantitis. The extracellular matrices of such biofilms contain polymeric substances, such as exopolysaccharides (EPS). The matrix produced by microorganisms can provide an essential scaffold for biofilm assembly. Additionally, it can promote microbial adhesion and cohesion while hindering diffusion, thereby making biofilms extremely difficult to treat or remove from surfaces.

The extracellular matrix contributes to the difficulty in the elimination of microbial biofilms within the oral cavity and human body, as well as on biomaterials, e.g., implants and medical devices, by antibodies, antibiotics and immune cells, which are largely unable to penetrate the dense extracellular matrix to kill the embedded microorganisms.

Biofilms on biological surfaces as well as on implants can be removed by chemical and/or mechanical cleaning and/or debridement.

### Debridement

Debridement is the medical removal of a patient's dead, damaged and/or infected tissue to improve the healing potential of the remaining healthy tissue. Debridement removal may be surgical, mechanical, chemical, autolytic (self-digestion), and by maggot therapy, wherein certain species of live maggots selectively eat only necrotic tissue.

Still, the mechanical cleaning and/or debridement of a surface, is not enough as it is not able to provide any secondary chemical and/or biological cleaning/decontamination effect, thus e.g. leaving the surface open for immediate repopulation of microbes, or even leaving traces of the prior microbial populations, e.g. on the inaccessible areas of rough surfaces.

Thus, there are several antimicrobial and/or anti-inflammatory agents used in anti-fouling treatments today.

### Hand sanitizers

A hand sanitizer is a liquid or gel generally used to decrease infectious agents on the hands. Formulations of the alcohol-based type are preferable to hand washing with soap and water in most situations in the healthcare setting. It is generally more effective at killing microorganisms and better tolerated than soap and water. They are typically available as liquids, gels, wipes and foams.

Alcohol-based versions typically contain some combination of isopropyl alcohol, ethanol (ethyl alcohol), or n-propanol. Versions that contain 60 to 95% alcohol are considered most effective. Care should be taken as they are flammable. Alcohol-based hand sanitizers works against a variety of microorganisms but not spores. Some versions contain compounds such as glycerol to prevent drying of the skin. Non-alcohol-based versions may contain benzalkonium chloride or triclosan.

Alcohol-based hand sanitizers have been commonly used in Europe since at least the 1980s. The alcohol-based version is on the World Health Organization's List of Essential Medicines, the safest and most effective medicines needed in a health system. The wholesale cost in the developing world is about US$1.40-3.70 per Liter bottle.

Hand sanitizers containing at least 60% alcohol or containing a "persistent antiseptic" should be used. Alcohol rubs kill many different kinds of bacteria, including antibiotic resistant bacteria and TB bacteria.

90% alcohol rubs are more effective against viruses than most other forms of hand washing. Isopropyl alcohol will also kill 99.99 % or more of all non-spore forming bacteria in less than 30 seconds, both in the laboratory and on human skin. But 90% alcohol rubs are highly flammable, but necessary to use to kill especially viruses, including enveloped viruses such as the flu virus, the common cold virus, coronaviruses, and HIV, though is notably ineffective against the rabies virus.

One well documented draw-back with commercially available hand-sanitizers is that the alcohol in hand sanitizers may not have the 10-15 seconds exposure time required to denature proteins and lyse cells. Further, it is often applied in too low quantities (0.3 ml) or in too low concentrations (alcohol concentrations below 60% will not kill virus and not harm many bacteria). In environments with high lipids or protein waste (such as food processing), the use of alcohol hand rubs alone may not be sufficient to ensure proper hand hygiene.

To solve this, there are new alcohol gel sanitizers marketed as alcohol rub sanitizers, which kill most bacteria, and fungi, and stop some viruses. Alcohol rub sanitizers containing at least 70% alcohol (mainly ethyl alcohol) kill 99.9% of the bacteria on hands 30 seconds after application and 99.99% to 99.999% in one minute. Still, they are notably less effective against virus.

### Hydrogen peroxide

Hydrogen peroxide (H₂O₂) is a very pale blue liquid which appears colourless in a dilute solution, slightly more viscous than water. It is a weak acid. It has strong oxidizing properties and is therefore a powerful bleaching agent that is mostly used for bleaching paper but has also found use as a disinfectant and as an oxidizer. Hydrogen peroxide in the form of carbamide peroxide is widely used for tooth whitening (bleaching), both in professionally- and in self-administered products.

Hydrogen peroxide is unstable and slowly decomposes in the presence of light. Because of its instability, hydrogen peroxide is typically stored with a stabilizer in a weakly acidic solution in a dark coloured bottle.

Hydrogen peroxide may be used for the sterilization of various surfaces, including surgical tools and may be deployed as a vapour (VHP) for room sterilization. H₂O₂ demonstrates broad-spectrum efficacy against virus, microbes including but not limited to bacteria, yeasts, and bacterial spores. In general, greater activity is seen against Gram-positive than Gram-negative bacteria though; however, the presence of catalase or other peroxidases in these organisms may increase tolerance in the presence of lower concentrations. Higher concentrations of H₂O₂ (10 to 30%v/v) and longer contact times are required for sporicidal activity.

Hydrogen peroxide is seen as an environmentally safe alternative to chlorine-based bleaches, as it degrades to form oxygen and water and it is generally recognized as safe as an antimicrobial agent by the U.S. Food and Drug Administration (FDA).

Historically, hydrogen peroxide was used for disinfecting wounds. Today, it is thought to inhibit healing and to induce scarring, because it destroys newly formed skin cells at high concentrations. One study found that only very low concentrations (0.03%v/v solution, this is a dilution of typical 3%v/v Peroxide by 100 times) may induce healing, and only if not applied repeatedly. A 0.5%v/v solution was found to impede healing.

### Pluronic acid

Pluronics® or poloxamers are tri-block copolymers of poly(ethylene oxide) poly(propylene oxide)-poly(ethylene oxide) (PEO-PPO-PEO). This group of synthetic polymers is thermo-reversible in aqueous solutions. The sol-gel transition is governed by the composition, molecular weight, and concentration of each constituent block polymer. The hydrophilic ethylene oxide and the hydrophobic propylene oxide give Pluronics an amphiphilic structure - meaning it has a polar, water-soluble group attached to a nonpolar water-insoluble hydrocarbon chain. Amphiphilic block copolymer molecules self-assemble into micelles (a packed chain of molecules) in aqueous solution. Micelle formation is temperature dependent and affects the degradation properties of the biomaterial: below a certain characteristic temperature known as the critical micelle temperature, both the ethylene and propylene oxide blocks are hydrated and the PPO block becomes soluble.

Pluronics can be found either as liquids, pastes or solids. Due to their amphiphilic characteristics (presence of hydrophobic and hydrophilic components), pluronics possess surfactant properties which allow them to interact with hydrophobic surfaces and biological membranes. Being amphiphilic also results in the ability of the individual block copolymers, known as unimers, to combine and form micelles in aqueous solutions. When the concentration of the block copolymers is below that of the critical micelle concentration (CMC), the unimers remain as molecular solutions in water. However, as the block copolymer concentration is increased above the CMC, the unimers will self-assemble and form micelles, which can take on spherical, rod-shaped or lamellar geometries. Their shapes depend on the length and concentration of the block copolymers (i.e. EO and PO), and the temperature. Micelles usually have a hydrophobic core, in this case the PO chains, and a hydrophilic shell, the EO chains.

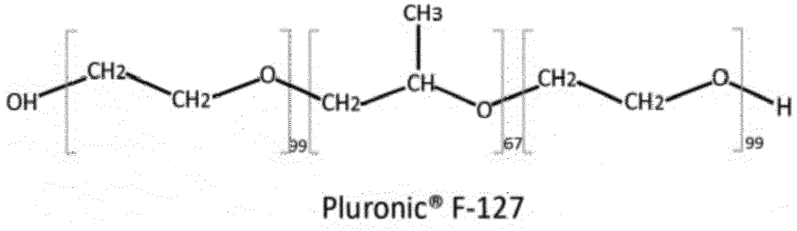

Pluronic F-127, also known as Poloxamer 407, is often used in tissue engineering because of the commercial availability of a consistent product that will undergo a sol-gel transition near physiological temperature and pH. A disadvantage of Pluronic F-127 is its fast degradation rate in vivo. To overcome this problem, Pluronic F-127 is frequently crosslinked with another α-hydroxy or amino acid in order to alter the chemical structure of its depsipeptide unit.

Pluronic acids form thermo-sensitive hydrogels, which are typically stabilized by addition of high-molecular-weight acids, such as hyaluronic acid.

Studies have documented the positive effects of pluronic acid formulations in reducing inflammation, protect tissues against damage and hinder microbial adhesion. Furthermore, it is EMA and FDA approved, completely biocompatible and safe to use clinically with no known harmful effects in human cells.

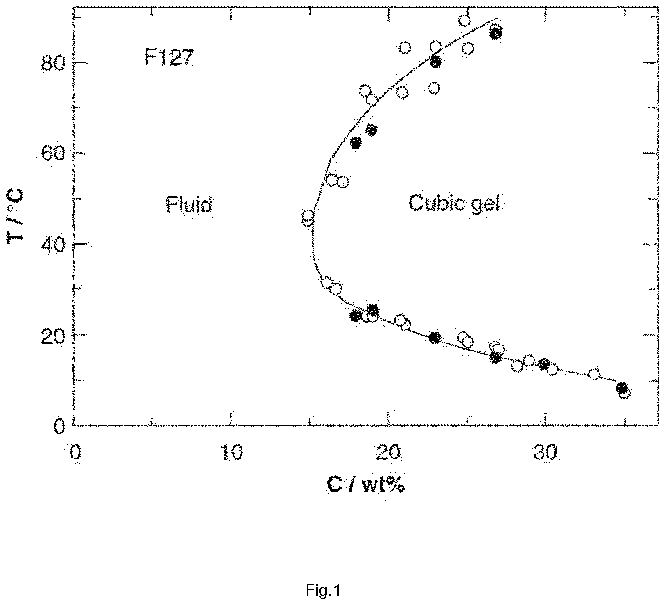

Still, it is hard to apply as a cleaning and debridement agent because it will automatically form a stable gel at over 18°C, or even between temperatures of 12-20°C (see figure 1) dependent of its concentration, which makes it unsuitable to use in small passages and/or for use on rough surfaces and does not lend it to application with a syringe.

Consequently, there is still sought for a means for cleaning and/or debriding fouled biological surface and/or a biomaterial surface *in situ,* or a means for preventing fouling of said biological surface and/or a biomaterial surface *in situ* by cleaning, sanitizing and/or sterilizing said surfaces e.g.in the oral cavity, or the exposed outer skin of the human body, such as for hand sanitizing.

The present invention presents an improved liquid composition for effectively cleaning, sanitizing and/or disinfecting a biological surface and/or a biomaterial surface *in situ.*

### SUMMARY

The present invention relates to an antimicrobial and/or anti-inflammatory composition for cleaning and/or sterilizing a biological surface and/or a biomaterial surface *in situ,* comprising at least two components
a. H₂O₂ at a final concentration of between 0.1 - 7%v/v, and
b. a composite hydrogel formulation comprising pluronic acid at a concentration of 0.1- 10%w/v.

Typically, an antimicrobial and/or anti-inflammatory composition according to the present invention comprises a composite hydrogel formulation of component b. which comprises pluronic acid at a concentration of 0.1-5%w/v, such as at a concentration of at the most 5%w/v, such as at a concentration of 0.1, 0.5, 1.0 or 1.5%w/v.

Typically, an antimicrobial and/or anti-inflammatory composition according to the present invention further comprises a H₂O₂ of component a. which has a final concentration of 0.1-7%v/v, such as a final concentration of 0,5-3,0 %v/v.

An antimicrobial and/or anti-inflammatory composition according to the present invention can further comprise water and/or physiological saline.

In one aspect, an antimicrobial and/or anti-inflammatory composition according to the present composition comprise the at least two components
a. H₂O₂, and
b. a composite hydrogel formulation comprising pluronic acid,
which are kept separate from each other until they are simultaneously mixed and applied to a biological surface and/or a biomaterial surface *in situ.*
According to that aspect, an antimicrobial and/or anti-inflammatory composition according to the present invention comprises a separate component a. which is a composition that comprises H₂O₂ at a concentration of at least 10-50%v/v.

A composition according to the present invention can further comprise a bioactive substance, which can be selected from the group consisting of EMD, peptides, drugs, bio active ions, small molecules, radioactive molecules, antimicrobial molecules and radio-opaque molecules.

A composition according to the present invention can further comprise a further antimicrobial substance.

A composition according to the present invention typically has a shelf-life of at least 1 years in RT.

A composition according to the present invention is intended for use in sterilizing a biological surface and/or a biomaterial surface *in situ.*

Typically, a composition according to the present invention is for use in cleaning skin and/or mucosa from microbes, wherein the microbes are typically selected from the group consisting of bacteria and virus, such as wherein the microbes are Riboviria, such as Coronaviridae, such as Orthocoronavirinae.

In a presently preferred aspect, a composition according to the present invention is for use in cleaning skin and/or mucosa from Coronavirus.

In consequence, the present invention relates to the application of an antimicrobial and/or anti-inflammatory composition according to the present invention as a hand wash, oral wash and/or for nasal and/or sinus cleansing, for washing e.g. but not limited to Coronavirus off skin and mucosa.

### DEFINITIONS and ABREVIATIONS

The term "microparticle" is herein meant to describe a particle having a mean particle diameter (D50) between about 1 and 1000 µm. Typically, in the present invention, a microparticle is used that has a mean particle diameter (D50) between 20-200 µm.

The present invention provides the means to clean and/or debride a medical and/or dental implant. In the present context, the term "implant" typically means a medical and/or dental implant.

In the present context, the term "dental implant" includes within its scope any device intended to be implanted into the oral cavity of a vertebrate animal, in particular a mammal such as a human, for example in tooth restoration procedures. Dental implants are herein selected from the group consisting of: Implants, bars, bridges, abutments, crowns, caps, and prosthetic parts in the oral cavity. Dental implants may also be denoted as dental prosthetic devices. Generally, a dental implant is composed of one or several implant parts. For instance, a dental implant usually comprises a dental fixture coupled to secondary implant parts, such as an abutment and/or a dental restoration such as a crown, bridge or denture. However, any device, such as a dental fixture, intended for implantation may alone be referred to as an implant even if other parts are to be connected thereto.

In the present context, the term "orthopaedic implant" includes within its scope any device intended to be implanted into the body of a vertebrate animal, in particular a mammal such as a human, for preservation and restoration of the function of the musculoskeletal system, particularly joints and bones, including the alleviation of pain in these structures. Non-limiting examples of orthopaedic implants are hip-joint prostheses, knee prostheses, elbow prostheses, finger prostheses, cochlear prostheses, and fixation screws.

In the present context, the term "vascular stent" refers to a tubular implant arranged for insertion into blood vessels of a vertebrate animal, in particular a mammal such as a human, in order to prevent or counteract a localized flow constriction, i.e. in order to counteract significant decreases in blood vessel diameter.

Hard tissues are, for example, bone, cementum, dentin, enamel, teeth, roots, cartilage and ligaments. Soft tissues are for example tissues that connect, support, or surround other structures and organs of the body, not being hard tissue such as bone. Soft tissue includes tendons, ligaments, fascia, skin, fibrous tissues, fat, and synovial membranes, muscles, nerves and blood vessels.

The term "debridement" in the present context means cleaning of a tissue surface, such as a surgically exposed hard and/or soft tissue surface, in order to remove, for example, biofilm, concrements, microbes, unwanted tissue, cells and cell residues, scar tissue, and/or necrotic tissue. Debridement may, for example, be performed in order to control and/or treat local infections, inflammations, foreign body reactions, pathological conditions, and/or regenerative processes (e.g. periodontitis, periimplantitis).

As used herein, a "biofilm" includes an extracellular matrix and one or more microorganisms such as, but not limited to, bacteria, fungi, algae and protozoa, which is attached to a surface. For example, but not by way of limitation, such surfaces can include tooth, mucosal, apatitic, bone and abiotic (e.g., implant, dentures, pipes, etc.) surfaces.

In the present context, the term "peroxide" is used interchangeably with Hydrogen peroxide (H₂O2).

A microorganism, or microbe, is a microscopic organism, which may exist in its single-celled form or in a colony of cells. Microorganisms include all unicellular organisms and so are extremely diverse. All of the Archaea and Bacteria are microorganisms (Prokaryotes). Some protists are related to animals and some to green plants. Many of the multicellular organisms are microscopic, namely micro-animals, some fungi and some algae.

An antimicrobial is an agent that kills microorganisms or stops their growth. Antimicrobial medicines can be grouped according to the microorganisms they act primarily against. For example, antibiotics are used against bacteria, and antifungals are used against fungi. They can also be classified according to their function. Agents that kill microbes are microbicidal, while those that merely inhibit their growth are called biostatic both are included in the term" antimicrobial". The use of antimicrobial medicines to treat infection is known as antimicrobial chemotherapy, while the use of antimicrobial medicines to prevent infection is known as antimicrobial prophylaxis.

A virus is a small infectious agent that replicates only inside the living cells of an organism. Viruses can infect all types of life forms, from animals and plants to microorganisms, including bacteria and archaea.

Antiviral drugs are a class of medication used specifically for treating viral infections rather than bacterial ones. Most antivirals are used for specific viral infections, while a broad-spectrum antiviral is effective against a wide range of viruses. Unlike most antibiotics, antiviral drugs do not destroy their target pathogen; instead they inhibit their development.

Antiviral drugs are one class of antimicrobials, a larger group which also includes antibiotic (also termed antibacterial), antifungal and antiparasitic drugs, or antiviral drugs based on monoclonal antibodies. Most antivirals are considered relatively harmless to the host, and therefore can be used to treat infections. They should be distinguished from viricides, which are not medication but deactivate or destroy virus particles, either inside or outside the body. Natural antivirals are produced by some plants such as eucalyptus and Australian tea trees.

As used in the present context, the term "antimicrobial", means that the composition is effective against microbes and virus. In its broadest meaning, the composition of the present invention is antimicrobial, i.e. it is antibacterial, antiviral, a bactericide and/or a viricide.

As used herein, the term "about" or "approximately" means within an acceptable error range for the particular value as determined by one of ordinary skill in the art, which will depend in part on how the value is measured or determined, i.e., the limitations of the measurement system.

### BRIEF DESCRIPTION OF THE DRAWINGS

- Figure 1:: Gel boundaries for aqueous saline (physiological conditions) solutions of copolymer F-127. The filled circles are data points obtained by the tube inversion method for the mixture. The unfilled squares are data points from rheometric analyses. The chart clearly indicates that Pluronic concentrations below 15% w/v in saline remains fluid independent of temperature. Fluid Pluronic solutions form independent micelles, but the concentration is not high enough for the Pluronic micelles to assemble into a cubic gel. Thus, low Pluronic concentrations that allow for micelle formation but stay liquid and act as detergent may be good for tissue-friendly cleaning of biological surfaces, but addition of other reactants that can contribute to the decontamination is expected to completely disrupt the micelle formation.

### DETAILED DESCRIPTION

The presently described composition is a long sought for means for effectively cleaning, disinfecting and/or sanitizing a biological surface and/or a biomaterial surface *in situ.* E.g., but not limited to a surface in the oral cavity for rapid and effective treatment essentially without leaving contaminating material residues, at the same time displaying an antimicrobial and/or anti-inflammatory effect.

The presently described composition is a water soluble and easy to rinse off, tissue-friendly non-ionic surfactant. It comprises a non-toxic formulation of well-studied active ingredients in clinical use which is particularly suitable for injectable, oral and cutaneous applications. The herein for the first time described compositions is proven to be non-sensitizing and non-irritating in clinical tests. The composition of the invention is compatible with other therapeutic agents against biofouling and inflammation.

The composition described herein has a liquid state at room temperature that makes for trouble-free mixing and application. It displays an easy flowing liquid consistence with surfactant effect that that allows the compositions to reach difficult places when applied into narrow defects and/or on skin.

The composition of the present invention mimics the natural release of reactive oxygen species (ROS) from peroxide produced by human cells. The charge from the reactive oxygen destroys microbial membranes and the oxygen itself is also toxic to anaerobic bacteria. The human cells themselves are protected against ROS by enzymes in their cell membrane and the local tissue can benefit from the increase in oxygen. Microbes have no such protection, nor can they develop resistance because of fundamental differences in their cell-membrane design. Thus, the composition can effectively dissolve biofilm, debris and mineral deposit as well as dissolve extracellular organics at the application site.

Moreover, the acidity of the peroxide helps cells dissolve mineral deposits as well as to break down and remove extracellular organics and calcifications from the contaminated implant.

Cells uses peroxide as a second messenger system that activate cellular defence against microbes. The presently disclosed composition mimics this signal to stimulate and strengthen the local cellular defence network.

Through the active oxygen released from the liquid hydrogel, the use of the herein presented composition also removes carbon contamination from a titanium containing implant surface and reactivates the titanium dioxide layer of the implant. This process re-establishes the original charge and hydrophilicity of the implant, restoring the optimal biological surface properties. This is a major factor for further survival, or even successful reintegration, of said treated implant. The effect is visualized in the experimental section by improved cell spreading and super-hydrophilicity.

The present composition is an advanced micelle forming gel formulation that works in synergy with natural occurring oxygen to break down and remove biofouling, eliminate microbes, keep tissue and implant moist and to reactivate the titanium implant surfaces

The hydrogel component of the composition and the active oxygen work in synergy to avoid foaming and keeps oxygen in place at the surface for a prolonged biological and chemical effect. The use of the composition provides moisture and allows the charged oxygen to work without risk of drying out tissue and/or implant, during which the active oxygen eradicates microbes that are then suspended and entrapped inside the hydrogel. Organic contaminants are in turn denatured by the strong detergent effect, broken down by the active oxygen and dissolved and entrapped in the hydrogel. Both hydrogel and oxygen reduce inflammation and support tissue health. The active oxygen released in turn strengthens the cellular defence network. In synergy, the gel and the active oxygen both removes contaminants and reactivates an implant surface. The inversed thermodynamics of the gel work with the disruptive effect of active oxygen to form an equilibrium between micelle-sol-state and gel-state that increases the debridement effect significantly.

The presently described composition is a novel formula of biocompatible hydrogel with strong, non-ionic detergent properties with improved sol-gel dynamics for solubilization and entrapment of debris and microbes for effectively cleaning and/or debriding a biological surface and/or a biomaterial surface *in situ.* It is easy rinsed off with water, and it completely decomposes to water, oxygen and carbon oxid.

The presently disclosed composition provides a novel and improved means for cleaning, santizing and/or disinfecting skin, for the treatment and elimination of biofilms; the prevention of biofilm formation; biofilm extracellular matrix degradation; and the inhibition of bacterial viability and growth within the biofilm. In particular, the presently disclosed subject matter provides a composition for the prevention and/or treatment of an oral disease (e.g., dental caries, periodontitis, gingivitis, mucositis and/or peri-implantitis). The invention itself is based on a combination of hydrogen peroxide (H₂O₂) and pluronic acid in such a concentration that the composition is in a liquid form . The H₂O₂ component of the composition can either be in the form of a concentrate (at a concentration of at least 10-50%v/v.) in a separate vial for mixing immediately before use in on embodiment applied with a mixing connector typically of Luer-lock design), or provided as dissolved directly into a hydrogel consisting of pluronic acid and water (or physiological saline) with a typical final concentration of 0.5 - 7%. The pluronic component itself can be any of the pluronic acids, e.g. the F-127 variety. The concentration of pluronic acid is typically 0.1-10% w/v, such as 0.1-2.5% w/v.

Pluronic-Acid works both as solubilizer and detergent in the composition disclosed herein, as well as a moisturizer and dynamic viscosity modifier. The hydrogel formulation establishes a dynamic equilibrium between sol-state and gel-state. This dynamic state facilitates efficient solubilization and entrapment of particles, microbes and pollutants during the debridement procedure.

The present innovation is based on a synergetic effect between pluronic acid and peroxide. Pluronic acid has a reversed thermodynamic ability to form a "packed" micelle structure with several micelles combining to form a hydrogel. This ability increases with increasing temperature and is shifted toward the gel state at physiological conditions (such as at >20 degrees Celsius). The peroxide addition allows the pluronic acid to stay in the liquid state (sol-state) at RT.

When mixed with hydrogen peroxide the present invention for the first time discloses that the sol-gel transition is more dynamic and less stable, so that the transition between sol-state (single solubilized micelles) and gel-state (packed micelle structure) is in a "dynamic" equilibrium with the peroxide radical activity even under physiological conditions (= high temperature). In effect this means that in the presence of peroxide, the packed micelle structure is dissolved and reforms constantly (not only by lowering or increasing temperature) also when the gel is applied onto human tissue/skin/mucosa. This effect increases the detergent and entrapment effect of the pluronic acid significantly.

Combined with the effect of hydrogen peroxide to release free oxygen radicals on viral particles, microbes and necrotic tissue, the sol-gel transitions dissolve and entrap organic contamination that are then removed when the gel is washed off.

Another advantage of the present composition is that the addition of hydrogen peroxide to the pluronic acid increases the temperature at when the packed micelle structure forms. I.e. that the pluronic gel, containing low concentrations of peroxide, is liquid at room temperate and thus can be applied through a syringe needle or from a dispenser bottle without clogging of the nozzle. This is not possible with pluronic acid alone because it starts forming a stable "packed micelle" gel already at room temperature and thus is very hard to squeeze through a narrow tip of a syringe, or a pump applicator from a dispenser bottle. Pluronic gel for wound care is therefore sold as gel in a box or a tube.

The increased effect from the combination of weak peroxide and pluronic acid was unforeseen and surprising.

The decreased viscosity of the pluronic hydrogel in combination with peroxide also assists during application. It enables the cleaning and/or debridement composition to reach narrow spaces and undercuts that a pluronic gel alone cannot get into because of its gel-state nature at physiological temperature. Thus, it is more efficient in cleaning rough (implant) surfaces, narrow spaces such as between bone and implant/tooth and in wrinkles on skin.

### Compositions

The present invention relates to a novel antimicrobial and/or anti-inflammatory composition for cleaning, sanitizing, sterilizing, disinfecting and/or debriding a biological surface and/or a biomaterial surface *in situ,* comprising at least two components
a. H₂O₂ at a final concentration of between 0.1 - 7%v/v, and
b. a composite hydrogel formulation comprising pluronic acid at a concentration of 0.1 - 10%w/v.

Said composition is liquid at room temperature, such as at a temperature of at the most 30°C, such as at a temperature between 20-30°C, such as at 25°C.

The presently disclosed composition is characterized in that it comprises a low concentration of pluronic acid, which allows the composition to be in a liquid state in room temperature instead of in a gel-state, which an essential feature for it to be usable for its intended purpose, cleaning, sanitizing, sterilizing, disinfecting and/or debriding a biological surface and/or a biomaterial surface *in situ.*

In particular, the liquid state (sol-state) of the hydrogel is essential for the intended use of the composition as a hand sanitizer, hand wash, oral wash and/or for nasal and/or sinus cleansing, in particular or washing virus off skin and mucosa.

The presently disclosed composition comprises the at least two components a. and b. in such a ratio that the composition is in a liquid state in room temperature instead of in a gel-state. Typical ratios of concentrations between component a. and component b. are approximately 1:1 (concentration of H₂O₂: concentration of pluronic acid). In general, the higher the concentration of the pluronic acid, the higher the concentration of the H₂O₂ is needed to keep the composition in a liquid state (Sol-state) at temperatures of between 20-30°C.

In one embodiment of an antimicrobial and/or anti-inflammatory composition according to the present invention, the composite hydrogel formulation of component b. comprises pluronic acid at a concentration of 0.1-10%w/v, such as at a concentration of 10%w/v, such as of 0.1, 0.5, 1, 1.5, 2, 2.5, 3, 3.5, 4, 4.5, 5, 5.5, 6, 6.5, 7, 7.5, 8, 8.5, 9 or 9.5 %w/v.

In another embodiment of an antimicrobial and/or anti-inflammatory composition according to the present invention, the composite hydrogel formulation of component b. comprises pluronic acid at a concentration of at the most 10%w/v, such as of at the most 0.1, 0.5, 1, 1.5, 2, 2.5, 3, 3.5, 4, 4.5, 5, 5.5, 6, 6.5, 7, 7.5, 8, 8.5, 9 or 9.5 %w/v.

An antimicrobial and/or anti-inflammatory composition according to the present invention can be a composition wherein the H₂O₂ of component a. has a final concentration of 0.1-7%v/v, such as 0.5-3%v/v, such as 0.1-5%v/v. In one embodiment, the H₂O₂ of component a. has a final concentration of no more than 7%v/v, such as 0.1-7%v/v, such as 1, 2, 3, 4, 5, 6 or 7%v/v.

An antimicrobial and/or anti-inflammatory composition according to the present invention can further comprise water and/or physiological saline.

The two components of the antimicrobial and/or anti-inflammatory composition according to the present invention can be in one solution or the at least two components can be kept separate from each other until they are simultaneously mixed and applied to a biological surface and/or a biomaterial surface *in situ.*

In an antimicrobial and/or anti-inflammatory composition according to the present invention, wherein the components are kept separate from each other before application, the separate component a. can be a composition that comprises H₂O₂ at a concentration of at least 10-50%v/v, such as at the most 10, 20, 30, 40 or 50%v/v. In one embodiment, a composition of the present invention comprises H₂O₂ at a concentration of 30%v/v.

### Emulsifier(s) and/or viscosity modifier(s)

In one embodiment, the antimicrobial and/or anti-inflammatory composition according to the present invention further comprises one or more emulsifier(s) and/or viscosity modifier(s). Said emulsifier and/or viscosity modifier may be selected from the group consisting of glycerine, glycols, polyethylene glycols (PEG), polyoxyethylene polyoxypropylene block copolymer (pluronic polyols), polyglycol alginate (PGA), CMC (carboxyl methyl cellulose), glycerol, Aloe Vera gel, alginate, hyaluronic acid (HA) and citosan.

The antimicrobial and/or anti-inflammatory composition according to the present invention may also comprise one or more detergent(s) selected from the group consisting of SDS (sodium dodecyl sulfate), sodium stannate, sodium pyrophosphate, oxine and SLS (sodium lauryl sulfate).

The antimicrobial and/or anti-inflammatory composition according to the invention may further comprise one or more flavouring oil(s), such as, but not limited to oils of spearmint, peppermint, wintergreen, sassafras, clove, sage, eucalyptus, marjoram, cinnamon and methyl salicylate and menthol.

The antimicrobial and/or anti-inflammatory composition according to the invention may further comprise one or more weak acidic buffers.

### Bioactive substance

Alternatively, or in addition, a composition according to the present invention can comprise a bioactive substance, typically selected from the group consisting of EMD, peptides, drugs, bio active ions, small molecules, radioactive molecules, antimicrobial molecules and radio-opaque molecules.

### Debridement components and antimicrobial substances

What is more, a composition according to the present invention can also comprise a further antimicrobial substance and/or debridement component.

In the present context, a further antimicrobial substance comprised in the composition according to the present invention can be selected from the non-exclusive list consisting of amoxicillin, doxycycline, cephalexin, ciprofloxacin, clindamycin, metronidazole, azithromycin, sulfamethoxazole and trimethoprim.

In one aspect, a further antimicrobial substance comprised in the composition according to the present invention is tetracycline, doxycycline, macrolides, penicillins (stabilized), chlorhexidine, chloramines and mixtures thereof.

In one aspect, a composition according to the present invention comprises a further anti-inflammatory substance.

### Shelf-life of at least 1 years in room temperature (RT)

A composition according to the present invention in one aspect has a shelf-life of at least 1 years in RT.

### A kit

The present invention also relates to a kit comprising a composition according to the present invention, comprising at least two containers comprising said separated components a. and b., respectively, a syringe and a vial, a connector device, an applicator tip and an instruction leaflet and optionally a mixing device and a debridement toll, such as but not limited to a brush. Said kit can provide the two components a. and b. in a two-chamber syringe, in which case the kit further can also comprise an instruction leaflet, a mixing device, an applicator tip and a debridement toll, such as but not limited to a brush a brush.

An antimicrobial and/or anti-inflammatory composition according to the present invention can be mixed before application and eventual storage or stored separately and mixed directly or shortly before and/or at the time of application. The application therefore, in another aspect is directed to a kit comprising a first container comprising component a), a second container comprising component b), and optionally at least one more (third or forth eytc.) container comprising a component c) (d) e) etc.) which can e.g. comprise microparticles and/or a mesh-forming substance and/or a bioactive substance and/or a debridement component and/or a further antimicrobial and/or anti-inflammatory substance.

Optionally such a kit may also comprise instructions for the preparation of the composition of the invention. The kit may also comprise one or more device(s) for the application of the composition to a subject. Such a device may e.g. be a syringe or an implant cleaning and/or debridement tool for cleaning and/or debriding an implant, such as in the oral cavity.

Preferably the implant cleaning and/or debridement tool comprises an elongated base member formed of at least two wires being twisted with each other, and a plurality of bristles fixed between said twisted wires and extending away from said twisted wires, whereby said bristles are positioned in a cleaning section at a first end of said base member; and that said bristles consist of titanium and/or a titanium alloy. A kit of the invention may also comprise the composition of the invention in one or more container(s) and an implant cleaning and/or debridement tool for cleaning and/or debriding an implant in the oral cavity.

One example of such an implant cleaning/debridement tool for cleaning a dental implant and/or debriding a hard tissue surface is disclosed in US 6,345,406, another example is given in WO 2009/083281.

The implant cleaning/debridement tool disclosed in WO 2009/083281 has bristles with diameters of 0.2 mm. In one aspect of the invention, a composition of the invention is particularly suitable to be used with this tool comprising solid microparticles in the composition of a size that will allow for an efficient cleaning of an implant and/or hard surface in the oral cavity. For this aspect, the microparticles optimally have a size about 150 pm, such as between 100 and 150pm, because the body tends to integrate particles in fibrous capsules when the particles are between 10-100 pm.

The present invention in one aspect thus relates to a kit comprising a composition according to any one of the preceding claims, comprising
a. at least two containers comprising said components a. and b., respectively,
b. a syringe and a vial,
c. a connector device,
d. an applicator tip, and
e. an instruction leaflet.

In another aspect, the present invention relates to a kit further comprising
f. a mixing device and
g. a debridement tool.

In a presently preferred aspect, a kit comprising a composition according to the present invention typically provides the two components a. and b. in a two-chamber syringe, further comprising an instruction leaflet, a mixing device, an applicator tip and optionally a debridement tool.

The kit can further comprise a container, such as a blister-pac and/or tissues, such as sanitizing wipes for applying the composition according to the present invention.

### Uses

The presently disclosed composition is intended for use in cleaning and/or debriding a biological surface and/or a biomaterial surface *in situ.*

In one aspect of the invention, the antimicrobial and/or anti-inflammatory composition according the present invention is for use in sterilizing, disinfecting and/or sanitizing a biological surface and/or a biomaterial surface in situ.

Thus, an antimicrobial and/or anti-inflammatory composition according to the present invention is typically used for cleaning, sterilizing, disinfecting and/or sanitizing skin and/or mucosa from microbes.

In particular, the microbes to be killed or removed by the composition according to the present invention are selected from the group consisting of bacteria and virus, such as selected from the non-exclusive group consisting of Riboviria, in particular Coronaviridae, such as but not limited to Orthocoronavirinae.

An antimicrobial and/or anti-inflammatory composition according to the present invention is in particular well-suited for killing and/or removing Coronaviruses, such as from skin and/or mucosa, including from hands.

The present invention thus in a presently preferred aspect also relates to the application of an antimicrobial and/or anti-inflammatory composition according to the present invention as a hand wash, oral wash, for nasal and/or sinus cleansing, and/or for washing virus off skin and mucosa.

The present invention thus in a presently preferred aspect also relates to the application of an antimicrobial and/or anti-inflammatory composition according to the present invention as a hand sanitizer, mouth wash, sinus rinse, nasal rinse and/or sanitizing wipes.

The presently disclosed composition is also useful for application in peri-implant defects, and the the formulation may be tailored to fit with various clinical procedures, such as but not limited to preventing periimplantitis, treating and/or preventing peri-implant mucositis, for peri-implant maintenance, peri-implant prophylaxis and post-operative follow-up administrations.

The presently disclosed composition can also be used for other oral procedures, such as during surgical debridement of periodontal defects, for preparation before regenerative procedures, in periodontal maintenance treatment, in periodontitis prophylaxis (dental hygienist), as well as in endodontics, both endodontally and in apical surgery.

What is more, the presently disclosed composition can further be used for cleaning and/or debriding outside the oral cavity, such as, but not limited to in orthopaedic revision surgery, debridement of transdermal devices, in dermal wound care for cleaning of acute wounds and/or in debridement of chronic ulcers and burn.

An antimicrobial and/or anti-inflammatory composition according to the present invention can typically be employed for use in the treatment and/or prevention of peri-implantitis, gingivitis and/or mucositis, peri-implant mucositis and/or periodontitis.

Periimplantitis is a typical complication related to orodental rehabilitation through the use of implants, i.e. a peri-implant disease, which is well-known to the person skilled in the art as an inflammatory reaction in which there is a loss of the bony support of the implant accompanied by inflammation. The aetiology of the disease is conditioned by the status of the tissue surrounding the implant, implant design, degree of roughness, the poor alignment of implant components, external morphology and excessive mechanical load.

The presently described antimicrobial and/or anti-inflammatory composition offers the means for an effective and rapid cleaning of an implant and/or for debriding a hard surface in the oral cavity essentially without damaging of the anatomical structure or of the implant and/or hard surface itself, and essentially without leaving contaminating material residues on the treated surface.

The invention therefore in one aspect is directed to the antimicrobial and/or anti inflammatory composition as defined herein and/or the kit for preparing the composition of the invention as defined herein, for use as a medicament.

Thus, the present invention relates to the use of an antimicrobial and/or anti-inflammatory composition according to the present invention for cleaning and/or debriding an implant in the oral cavity, such as an implant *in situ,* a hard surface in the oral cavity, such as an outer surface of a hard tissue in the oral cavity, a surgically exposed hard surface in the oral cavity, a wound in the oral cavity, such as a wound resulting from periimplantitis or a surgical wound, a periodontal defect and/or periodontal wound, and/or an oral hard tissue defect.

### Implants

The invention also relates to the use of the antimicrobial and/or anti-inflammatory composition as defined herein and/or the kit for preparing the composition of the invention as defined herein, for the preparation of a medicament and/or a pharmaceutical and/or cosmetic composition, for cleaning and/or debriding an implant in the oral cavity, such as an implant *in situ,* a hard surface in the oral cavity, such as an outer surface of a hard tissue in the oral cavity, a surgically exposed hard surface in the oral cavity, a wound in the oral cavity, such as a wound resulting from periimplantitis or a surgical wound, a periodontal defect and/or periodontal wound, and/or an oral hard tissue defect.

The invention is also directed to the antimicrobial and/or anti-inflammatory composition as defined herein or the kit for preparing the composition of the invention as defined herein for use for cleaning and/or debriding an implant in the oral cavity, such as an implant *in situ,* a hard surface in the oral cavity, such as an outer surface of a hard tissue in the oral cavity, a surgically exposed hard surface in the oral cavity, a wound in the oral cavity, such as a wound resulting from periimplantitis or a surgical wound, a periodontal defect and/or periodontal wound, and/or an oral hard tissue defect.

Another presently preferred embodiment is directed to the use of an antimicrobial and/or anti-inflammatory composition according to the present invention together with an implant cleaning and/or debridement tool, for cleaning an implant and/or debriding a hard surface in the oral cavity. Said implant cleaning and/or debridement tool is e.g. characterized by comprising an elongated base member formed of at least two wires being twisted with each other, and a plurality of bristles fixed between said twisted wires and extending away from said twisted wires, whereby said bristles are positioned in a cleaning section at a first end of said base member; and that said bristles comprise or consist of titanium and/or a titanium alloy.

Many medical implants, such as e.g. dental implants, orthopaedic implants and vascular stents, are metallic, i.e. they are made of a metal material. The present invention consequently relates to the use of an antimicrobial and/or anti-inflammatory composition according to the present invention, alternatively together with an implant cleaning and/or debridement tool, for cleaning and/or debriding an implant made of a metal material. Examples of metal materials commonly utilized for constructing metallic medical implants are steel, titanium, zirconium, tantalum, niobium, hafnium and alloys thereof. In particular, titanium and titanium alloys have proven to be suitable to utilize for constructing medical implants.

On the other hand, both medical and dental implants can at least partially, as well as in full (full-ceramic implants) consist of porcelain and/or ceramic, such as of zirconium oxide and/or hydroxyapatite, or any other ceramic or porcelain material known to the person skilled in the art as being suitable for implantry. Thus, the present invention equally relates to the use of an antimicrobial and/or anti-inflammatory composition according to the present invention, alternatively together with an implant cleaning and/or debridement tool, for cleaning and/or debriding an implant made of, or comprising, porcelain and/or ceramic. Consequently, the present invention is also directed to the use of a composition of the invention for the preparation of a medicament and/or a pharmaceutical and/or cosmetic composition for the cleaning and/or debridement of an implant made of or comprising porcelain and/or ceramic. Also, the invention is directed to a composition of the invention alternatively for use for cleaning and/or debriding an implant made of or comprising porcelain and/or ceramic.

Dental implants are typically utilized in dental restoration procedures in patients having lost one or more of their teeth. A dental implant comprises a dental fixture, which is utilized as an artificial tooth root replacement. Thus, the dental fixture serves as a root for a new tooth. The dental fixture is typically a screw, i.e. it has the shape of a screw, and it is typically made of titanium, a titanium alloy, zirconium or a zirconium alloy. The screw is surgically implanted into the jawbone, where after the bone tissue grows around the screw and the screw is fixated in the bone with the bone in close contact with the implant surface. Once the implant screw is firmly anchored in the jawbone, it may be elongated by attachment of an abutment to the screw. The abutment may, just as the screw, be made of titanium, a titanium alloy, zirconium or a zirconium alloy. The shape and size of the utilized abutment are adjusted such that it precisely reaches up through the mucosa after attachment to the screw. A dental restoration such as a crown, bridge or denture may then be attached to the abutment. Alternatively, the implant screw has such a shape and size that it reaches up through the mucosa after implantation, whereby no abutment is needed and a dental restoration such as a crown, bridge or denture may be attached directly to the screw.

The present invention consequently relates to the use of an antimicrobial and/or anti-inflammatory composition according to the present invention, alternatively together with an implant cleaning and/or debridement tool, for cleaning and/or debriding any parts of a dental implant, selected from the group consisting of dental fixture such as a screw, abutment, and dental restoration such as a crown, bridge or denture. Consequently, the present invention is also directed to the use of a composition of the invention for the preparation of a medicament and/or pharmaceutical and/or cosmetic composition for cleaning and/or debriding any parts of a dental implant, selected from the group consisting of dental fixture such as a screw, abutment, and dental restoration such as a crown, bridge or denture. Also, the invention is directed to a composition of the invention for use for cleaning and/or debriding any parts of a dental implant, selected from the group consisting of dental fixture such as a screw, abutment, and dental restoration such as a crown, bridge or denture.

The present invention further relates to the use of an antimicrobial and/or anti inflammatory composition according to the present invention, alternatively together with an implant cleaning and/or debridement tool, for cleaning and/or debriding orthopaedic implants, such as orthopaedic implants which are utilized for the preservation and restoration of the function in the musculoskeletal system, particularly joints and bones, including alleviation of pain in these structures, and/or for cleaning and/or debriding vascular stents, i.e. tubular implants arranged for insertion into blood vessels in order to prevent or counteract a localized flow constriction. Consequently, the present invention is also directed to the use of a composition of the invention for the preparation of a medicament for cleaning and/or debriding orthopaedic implants, such as orthopaedic implants which are utilized for the preservation and restoration of the function in the musculoskeletal system, particularly joints and bones, including alleviation of pain in these structures, and/or for cleaning and/or debriding vascular stents. Also, the invention is directed to a composition of the invention for use for cleaning and/or debriding orthopaedic implants, such as orthopaedic implants which are utilized for the preservation and restoration of the function in the musculoskeletal system, particularly joints and bones, including alleviation of pain in these structures, and/or for cleaning and/or debriding vascular stents.

The surface of medical implants such as e.g. dental implants, orthopaedic implants and vascular stents, or the vicinity thereof, has sometimes to be cleaned after placing. This is particularly important when an infection or contamination occurs, causing a progressive degenerative process in the bone adjacent to the implant known as periimplantitis. In these cases, the surface of the ailing implant has to be cleaned from microbes and contaminants to stop the progression of the disease and ensure re-integration of the implant. Failure to clean the implant surface will eventually lead to loss of bone and implant and make further alternative treatments difficult and sometimes even impossible. Furthermore, the surface of vascular stents may have to be cleaned during implantation in order to remove coagulum, and the interior of vascular stents, i.e. the cavity within vascular stents, may have to be cleaned in an endoscopic procedure during a later treatment due to restenosis, i.e. blocking of the blood vessel.

The present invention therefore relates to the use of an antimicrobial and/or anti-inflammatory composition according to the present invention, alternatively together with an implant cleaning and/or debridement tool, for cleaning and/or debriding an implant or the vicinity thereof after placing. Consequently, the present invention is also directed to the use of a composition of the invention for the preparation of a medicament for cleaning and/or debriding an implant or the vicinity thereof after placing. Also, the invention is directed to a composition of the invention for use for cleaning and/or debriding an implant or the vicinity thereof after placing.

In addition, for different reasons, it may be advantageous or necessary to debride surgically exposed hard tissue surfaces. For example, debriding of surgically exposed hard tissue surfaces may be advantageous or necessary to perform before regenerative treatment, i.e. in order to prepare the hard tissue surfaces for regenerative treatment. Examples of conditions, which may be associated with a treatment in which debridement of a surgically exposed hard tissue surface is advantageous or necessary to perform in order to prepare the surface for regenerative treatment, are: periimplantitis, periodontitis lesions, marginal periodontitis, apical periodontitis, furcation defects, apical granulomas and cysts, bone cysts, bone tumors, bone granulomas, bone cancers, (infected) extraction sockets, alveolitis sicca ("dry socket"), cleaning of apicectomy defects, localized osteomyelitis, trauma induced defects, resection or revision of implants, resection or revision of fractures, and removal of temporary bone implants (such as orthopaedic bone plates, retainers and screws). Furthermore, debridement of articular surfaces in joints affected by arthritis and debridement of such surfaces before regenerative treatment for cartilage and ligaments is instituted may also be advantageous or necessary to perform.

The present invention thus relates to the use of an antimicrobial and/or anti-inflammatory composition according to the present invention, alternatively together with an implant cleaning and/or debridement tool, for cleaning and/or debriding surgically exposed hard tissue surfaces before regenerative treatment. Consequently, the present invention is also directed to the use of a composition of the invention for the preparation of a medicament and/or a pharmaceutical and/or cosmetic composition for cleaning and/or debriding surgically exposed hard tissue surfaces before regenerative treatment. Also, the invention is directed to a composition of the invention for use for cleaning and/or debriding surgically exposed hard tissue surfaces before regenerative treatment.

The antimicrobial and/or anti-inflammatory composition according to the present invention, alternatively together with an implant cleaning and/or debridement tool, may be utilized during surgery for cleaning of the surface of a metallic medical implant after infection and/or bone resorption. For example, it may be utilized for cleaning the surface of a metallic dental implant and/or a metallic orthopaedic implant. Thus, it may be utilized for removing e.g. bacterial biofilm, debris, calculus or fibrous tissue from the surface of a dental implant, such as a titanium screw. Alternatively, it may be utilized together with a further cleaning agent (i.e. an antibacterial agent) in order to remove the bacterial biofilm from the vicinity of the dental fixture during implantation. It may also be utilized for cleaning the surface of, or the vicinity of, an abutment. Consequently, the present invention is also directed to the use of a composition of the invention for the preparation of a medicament for cleaning, e.g. removing bacterial biofilm, debris, calculus or fibrous tissue from the surface of a metallic dental implant, such as a titanium screw or an abutment, or a metallic orthopaedic implant. Also, the invention is directed to a composition of the invention for use for cleaning, e.g. removing bacterial biofilm, debris, calculus or
fibrous tissue the surface of a metallic dental implant, such as a titanium screw or an abutment, or a metallic orthopaedic implant.

In addition, the antimicrobial and/or anti-inflammatory composition according to the present invention, alternatively together with an implant cleaning and/or debridement tool, may be utilized for removing cement remnants, bacterial biofilm, debris, calculus or fibrous tissue from the surface of an orthopaedic implant or for removing plaque from the surface of a vascular stent. Alternatively, it may be utilized for cleaning the interior of a vascular stent, i.e. the cavity within a vascular stent, in an endoscopic procedure during a later treatment due to restenosis, i.e. blocking of the blood vessel.

A procedure involving use of the antimicrobial and/or anti-inflammatory composition according to the present invention, alternatively together with an implant cleaning and/or debridement tool, may, for example, involve the steps of: surgically exposing a hard tissue surface to be treated; removal of inflamed soft tissue; debriding the surface by means of applying the antimicrobial and/or anti-inflammatory composition according to the present invention, alternatively together with an implant cleaning and/or debridement tool; applying (regenerative) treatment as needed; replacing soft tissue; suturing for good primary closure and wound stability; and allowing the wound to heal.

In particular, the antimicrobial and/or anti-inflammatory composition according to the present invention, alternatively together with an implant cleaning and/or debridement tool, is an efficient tool for debridement of surgically exposed tooth root surfaces, furcation defects and bony defects before regenerative treatment (i.e. by means of, for example Straumann® Emdogain, bone graft materials, autologous bone, membranes, etc.), the antimicrobial and/or anti-inflammatory composition according to the present invention, alternatively together with an implant cleaning and/or debridement tool, is especially effective for removing granulation tissue, and for removing concrements of calcified biofilms (plaques) and subgingival calcus.

The antimicrobial and/or anti-inflammatory composition according to the present invention, alternatively together with an implant cleaning and/or debridement tool, is advantageous to utilize for cleaning and/or debriding both "hard" metallic medical and/or dental implants having relatively hard surfaces, such as e.g. medical implants of steel, and "soft" metallic medical implants having delicate surfaces, such as e.g. medical and/or dental implants of titanium, a titanium alloy, zirconium or a zirconium alloy.

In addition, the antimicrobial and/or anti-inflammatory composition according to the present invention does not leave contaminants, i.e. material residues, incompatible with reintegration of the implanted structure. Thus, the inflammation risk is minimal.

In particular, a relatively rapid debridement procedure of surfaces, which are otherwise hard to clean and/or hard to reach by hand instrumentation, may be performed by means of the antimicrobial and/or anti-inflammatory composition according to the present invention, alternatively together with an implant cleaning and/or debridement tool. Rapid treatment ensures a better treatment outcome. As mentioned above, it is a well-known fact that the morbidity and frequency of adverse effects, such as e.g. post-surgery effects, are directly related to, and often proportional to, the time used for the debridement of surgically exposed hard tissue surfaces. Thus, rapid debridement treatment ensures a better total treatment outcome.

The use of the antimicrobial and/or anti-inflammatory composition according to the present invention, alternatively together with an implant cleaning and/or debridement tool, is especially favorable where the treatment plan for a defect includes placing of a titanium implant or any other device made of titanium, since only titanium and no other metallic ions or polymers that can provoke unwanted and/or adverse clinical and/or biological effects can contaminate the treated area, hampering the outcome of planned and/or future implant procedures.

### Oral hygiene

In oral hygiene and dentistry, debridement refers to the removal of plaque and calculus that have accumulated on the teeth, which can be performed routinely by the technician, for medical, hygienic, as well as for purely cosmetic reasons. Thus, in one embodiment, the antimicrobial and/or anti-inflammatory composition according to the present invention, again alternatively together with an implant cleaning and/or debridement tool, is used for removal of plaque and calculus that have accumulated on the patient's natural teeth, or tooth implants. The antimicrobial and/or anti-inflammatory composition according to the present invention comprises radicalized oxygens and is thus particularly suitable for use in the bleaching of natural and/or artificial teeth.

### Microorganisms

An antimicrobial and/or anti-inflammatory composition according to the present invention is in general intended for use in cleaning, disinfecting, sanitizing and/or debriding a biological surface and/or a biomaterial surface *in situ,* e.g. for use in removal of biofouling, biofilm and/or necrotic tissue from such a biological surface and/or a biomaterial surface *in situ.*

Biofilms that can be prevented, eliminated and/or treated by the composition of the present disclosure include, but are not limited to, biofilms present within the oral cavity, e.g., on the surface of teeth, on the surface of mucosal/soft-tissues such as gingivae/periodontium and inside a tooth canal (e.g. the endodontic canal).

In certain embodiments, biofilms that can be prevented, eliminated and/or treated by the composition of the present disclosure include biofilms on the urinary tract, lung, gastrointestinal tract, on and/or within chronic wounds, and present on the surface (e.g., implants) and within medical devices and medical lines, e.g., catheters, medical instruments and medical tubing.

The composition of the present disclosure can be used to reduce the growth and/or inhibit the viability of one or more microorganisms, e.g., bacteria in a biofilm. For example, and not by way of limitation, the bacteria can include *Streptococcus mutctns (S. mutctns), Streptococcus sobrinus, Streptococcus sctnguis (sctnguinis), Streptococcus gordonii, Streptococcus omlis, Streptococcus mitis, Actinomyces odontolyticus, Actinomyces viscosus, Aggregcttibctcter ctctinomycetemcomitctns, Ictctobctcillus spp., Porphyromoncts gingivctlis, Prevotellct intermedia, Bacteroides forsythus, Treponema denticola, Fusobacterium nucleatum, Campylobacter rectus, Eikenella corrodens, Veillonella spp., Micromonas micros, Porphyromonas cangingivalis, Haemophilus actinomycetemcomitans Actinomyces spp., Bacillus spp., Mycobacterium spp., Fusobacterium spp., Streptococcus spp., Staphylococcus aureus, Streptococcus pyogenes, Streptococcus agalectiae, Proteus mirabilis, Elebsiella pneumoniae, Acinetobacter spp., Enterococcus spp., Prevotella spp., Porphyromonas spp., Clostridium spp., Stenotrophomonas maltophilia, P. cangingivalis, Candida albicans, Escherichia coli* and/or *Pseudomonas aeruginosa.* In certain embodiments, the bacteria are *S*. *mutans,* which is present within biofilms found in the oral cavity, e.g., on the surface of teeth.

The microorganisms most commonly associated with implant failure are spirochetes and mobile forms of Gram-negative anaerobes. Diagnosis can be based on changes of colour in the gum, bleeding and probing depth of peri-implant pockets, suppuration, x-ray and gradual loss of bone height around the tooth. The antibiotic therapy proven to be most efficacious in the antibiogram has so far been the association of amoxycillin and clavulanic acid. In addition to bacterial infections, microbial infections in the oral cavity can of course also include fungal and/or viral infections.

An antimicrobial and/or anti-inflammatory composition according to the present invention is effective for killing bacteria, fungus and/or virus.

What is more, the composition described herein is antimicrobial, without causing microbial resistance, as well as anti-inflammatory.

In consequence, the present invention relates to a method for treating and /or preventing periimplantitis, gingivitis and/or mucositis, peri-implant mucositis and/or periodontitis, comprising cleaning and/or debriding a biological surface and/or a biomaterial surface *in situ by applying* a composition according to the present invention to said fouled, filmed and/or necrotized surface.

### EXAMPLES

The following Examples have been included to provide guidance to one of ordinary skill in the art for practicing representative embodiments of the presently disclosed subject matter. Considering the present invention and the general level of skill in the art, those of skill can appreciate that the following Examples are intended to be exemplary only and that numerous changes, modifications, and alterations can be employed without departing from the scope of the presently disclosed subject matter.

### Example 1

### Analysis of micelle formation of Pluronic in presence of peroxide

Pluronic® F-127 added to an aqueous solution can change its thickness depending on the concentration and temperature. F-127 is a non-ionic, surfactant polyol with a molecular weight of 12500 Daltons.

As can be seen in figure 1, gel boundaries for aqueous saline (physiological conditions) solutions of copolymer F-127. The filled circles are data points obtained by the tube inversion method for the mixture. The unfilled squares are data points from rheometric analyses. The chart clearly indicates that Pluronic concentrations below 15% w/v in saline remains fluid independent of temperature. Fluid Pluronic solutions form independent micelles, but the concentration is not high enough for the Pluronic micelles to assemble into a cubic gel. Thus, low Pluronic concentrations that allow for micelle formation but stay liquid and act as detergent may be good for tissue-friendly cleaning of biological surfaces, but addition of other reactants that can contribute to the decontamination is expected to completely disrupt the micelle formation.

### Adding Peroxide to the Pluronic f-127 gel

In order to test if micelle formation (as visualised by ability to foaming and dissolve oil) still could be obtained when mixing low concentration of Pluronic f-127 in water with hydrogen peroxide, in a wide range of peroxide concentrations, was tested (Table 1).

The technique used for making aqueous poloxamer solution is simple. Weighed amount of the f-127 is slowly added to a known weight of cold water (less than 10 °C) with careful stirring. The stirring rate should be controlled so as to maintain a slight vortex in the liquid. Too rapid a stirring rate will cause aeration and the formation of foam.

| **Sample** | **Pluronic F-127(gr)** | **H₂O (ml)** | **50% H₂O₂(ml)** |
|---|---|---|---|
| *0* | 2,5 | 50,00 | 0,00 (0,0%) |
| *1* | 2,5 | 49,90 | 0,10 (0,1%) |
| *2* | 2,5 | 49,50 | 0,50 (0,5%) |
| *3* | 2,5 | 49,00 | 1,00 (1,0%) |
| *4* | 2,5 | 48,50 | 1,50 (1,5%) |
| *5* | 2,5 | 47,00 | 3,00 (3,0%) |
| *6* | 2,5 | 44,00 | 6,00 (6,0%) |
| *7* | 2,5 | 38,00 | 12,00 (12,0%) |

Table 1: Compositions of suspensions containing 5% Pluronic Acid F-127 (Zigma Aldrich) in water and a range of Hydrogen Peroxide (Zigma Aldrich). After dissolving the f-127 in cold water, the right amount of hydrogen peroxide was added, mixing was done by inverting tubes carefully to avoid foaming, and the solutions were left on the bench at room temperature for 10 minutes. When all the polymer has been added, stirring can be continued (while keeping the solution cool) until a clear solution is formed or the container can be placed in a refrigerator and left undisturbed for several hours, at which time solution is complete. The H2O2 was mixed in by slight stirring for a few seconds when the experiment started.

| **Sample** | **Foaming (0-3)** | **Oil absorbtion per 50 ml (droplets)** | **% of H₂O₂** |
|---|---|---|---|
| *0* | 3 | 7 | 0,00 |
| *1* | 2 | 6 | 0,1% |
| *2* | 3 | 12 | 0,5% |
| *3* | 3 | 11 | 1,0% |
| *4* | 3 | 7 | 1,5% |
| *5* | 2 | 5 | 3,0% |
| *6* | 1 | 2 | 6,0% |
| *7* | 1 | 0 | 12,0% |

Table 2: After incubation all the solutions were still liquid (no gel formation as expected) and were vortexed and the ability to form foam (as a simple measurement of surfactant effect) was scored on a scale from 0 (no foam) to three (much foam). As expected, sample 0 scored maximum, and addition of hydrogen peroxide reduced foaming at low and high concentration. Unexpectedly there were no linearity in the reduction of foaming. In fact, samples 2 trough 4 scored as high as the control, indicating that micelle formation and surfactant effect were preserved in these samples. The samples were then tested on how many oil droplets (mineral oil stained with oil red (Zigma Aldrich)) from a needle point the solutions could absorbed before getting turbid. Turbidity is a sign of macro-emulsion, a stage where oil droplets become so big that they disperse light. The positive control, Sample 0, absorbed 7 oil droplets before getting turbid. The negative control, sample 7, turned turbid with the first droplet. The expected linearity between these extremes was however not observed. Surprisingly, sample 2 (0,5% Peroxide) and 3 (1,0% peroxide) actually absorbed almost twice as many droplets than the positive control before turbidity was observed. This indicate that Pluronic f-127 micelle formation at low concentrations (=liquid solutions) actually is more efficient, and dynamic, in presence of low concentrations of hydrogen peroxide in the range of 0,5 - 1,5% volume by volume (in a 5% w/v solution of f-127).

### EXAMPLE 2

### Test of biocompatibilty of Pluronic acid combined with peroxide

### Test procedure:

The biocompatibility test was performed according to ISO 10993-5:2009 Biological evaluation of medical devices Part 5: Tests for in vitro cytotoxicity.

Colorimetric assay such MTT, WST-1 or LDH was not used as the peroxide suspension degrade the indicator colour used in these assays and thus, they cannot be used. Instead radioactive labelling with [3H] thymidine incorporation was used to analyse the cytotoxicity of the Pluronic-peroxide solution on normal human dermal fibroblast cells (NHDF) (Lonza Walkersville, Inc. Walkersville, MD, USA).

Due the viscosity of the tested hydrocolloid gels (SilvaSorb® Gel, Medline Industries, Munedelein, IL) and the Pluronic-Peroxide gels (20%Pluronic f-127 in water with 3% Hydrogen Peroxide), the test samples could not be diluted into the cell culture medium. Nor could the NHDF cells grow directly onto the gel due their lack of surface properties. To overcome this problem, the NHDF cells were grown on cell culture inserts (Millicell®, Millipore Corp., Billerica, MA, USA) that was inserted into a well that contained the test gel diluted 1:1 against cell culture medium. The NHDF cells were cultivated in 24 well plates for 24 hours at 37C and 5% CO2 with Dulbecco's PBS cell culture media (FGM®-2 with Insulin (CC-4021J), rhFGF-B (CC-4065J) and FBS (CC-4161J), Lonza Walkervill, MD USA). After 24 hours growing in presence of test substances or controls (no gel and gelatin gel) 0.5 µL 3H-thymidine (PerkinElmer, Boston, USA) was added into the wells containing the cells. After 12 hours of exposure to thymidine the cells were washed three times in cold PBS and lysed with 250µL 1M NaOH. Then 200µL of the solubilized cell solution was transferred to 3ml lnsta-gel-2-Pluss liquid scintillation fluid. Subsequently the scintillations were counted in a liquid scintillation analyzer (TRI-Corb® 1500 Perkin Elmer).

Results and conclusion: There were no observed difference in count number between samples exposed to Pluronic-Peroxide solution and the controls indicating that the NHDF cells grew at normal rate in presence of these test gel. The SilvaSorb gel showed a significant decrease in count number indication slower cell growth in presence of this gel as has also been repeatedly reported in the scientific literature, indicating that this gel is slightly toxic NHDF cells.

### EXAMPLE 3

### Chemical cleaning efficacy of liquid Pluronic f-127 solutions containing peroxide

The following cleaning solutions were tested:

| Negative control | Brain Heart Infusion Broth (BHI broth) |
|---|---|
| Test 1 | H2O2 3% v/v + f-127 1% w/v |
| Test 2 | H2O2 5% v/v + f-127 1% w/v |
| Test 3 | H2O2 3% v/v + f-127 7% w/v |
| Test 4 | H2O2 5% v/v + f-127 7% w/v |
| Test 5 | Chlorhexidine 0,2% w/v + f-127 7% w/v |
| Test 6 | Ethanol 75% v/V + f-127 1 % w/v |

### Experimental set-up:

A stock *Streptococcus Epidermidis* culture was established in BHI broth and allowed to grow to log phase.

The S. Epidermidis culture was introduced to cell culture disks of c.p. titanium and allowed to for a multilayer biofilm on the surface over a period of 2 days. After the biofilm had been established, the discs were rinsed several times in sterile, cold PBS until only adherent cells was present on the surfaces.

The discs with biofilm on were then submerged in test gel or control for 5 minutes ± 5 sec. making sure that the entire discs were covered by the test gels all the time. The discs were then rinsed for 10 minutes in cold PBS on a shaker, rinsed off and viewed in a tabletop scanning electron microscope to see how many bacteria that were left on the test surfaces.

### Results and conclusion:

The study showed that cleaning efficacy increased some with increasing H2O2 and increasing Pluronic f-127 concentration. The difference between test 1 and test 4 was however surprisingly small and not statistically significant, as all four tests performed very well and almost no bacteria remained on the surface. The negative control was as expected completely covered by bacteria with no effect of the PBS washing. The positive Chlorhexidine and Ethanol controls was surprisingly ineffective. Almost all of the biofilm was retained on the surfaces in these two groups. Later analysis with regrowth of bacteria from these surfaces revealed that all bacteria in these two tests were dead, but that the bulk of the biofilm remained attached to the surface, forming a contaminated layer that provide a strong bridgehead for new biofilm formation if a new contamination occurs. The surprising finding in this study is that it seems to be a synergistic effect between hydrogen peroxide and Pluronic f-127 that not only kills bacteria but also disintegrate and solubilize the entire biofilm, leaving an almost totally decontaminated surface. The concentration of the peroxide and Pluronic can be reduced toward 3% hydrogen peroxide in 1% Pluronic acid without losing much effect compared to stronger solutions. This also indicate that the synergy between hydrogen peroxide and Pluronic acid can be fine-tuned and probably has an optimal concentration-relation that allow for rapid and dynamic micelle (detergent) effect and at the same time maintains the effectiveness of activated oxygen release that kill bacteria and dissolves organic molecules. This optimal formulation appears to be quite low, and much lower than what one would expect based on analysis of the separate ingredients.

## Claims

1. An antimicrobial and/or anti-inflammatory composition for cleaning and/or sterilizing a biological surface and/or a biomaterial surface *in situ,* comprising at least two components
c. H₂O₂ at a final concentration of between 0.1 - 7%v/v, and
d. a composite hydrogel formulation comprising pluronic acid at a concentration of 0.1- 10%w/v.

2. An antimicrobial and/or anti-inflammatory composition according to claim 1, wherein the composite hydrogel formulation of component b. comprises pluronic acid at a concentration of 0.1-5%w/v.

3. An antimicrobial and/or anti-inflammatory composition according to claim 2, wherein the composite hydrogel formulation of component b. comprises pluronic acid at a concentration of at the most 5%w/v.

4. An antimicrobial and/or anti-inflammatory composition according to claim 2, wherein the composite hydrogel formulation of component b. comprises pluronic acid at a concentration of 0.1, 0.5, 1.0 or 1.5%w/v.

5. An antimicrobial and/or anti-inflammatory composition according to any of the preceding claims, wherein the H₂O₂ of component a. has a final concentration of 0.1-7%v/v.

6. An antimicrobial and/or anti-inflammatory composition according to any of the preceding claims, wherein the H₂O₂ of component a. has a final concentration of 0,5-3,0 %v/v.

7. An antimicrobial and/or anti-inflammatory composition according to any of the preceding claims, further comprising water and/or physiological saline.

8. An antimicrobial and/or anti-inflammatory composition according to any of the preceding claims, wherein the at least two components
a. H₂O₂, and
b. a composite hydrogel formulation comprising pluronic acid, are kept separate from each other until they are simultaneously mixed and applied to a biological surface and/or a biomaterial surface *in situ.*

9. An antimicrobial and/or anti-inflammatory composition according to claim 5, wherein the separate component a. is a composition that comprises H₂O₂ at a concentration of at least 10-50%v/v.

10. A composition according to any one of the preceding claims, further comprising a bioactive substance.

11. A composition according to claim 10, wherein the bioactive substance is selected from the group consisting of EMD, peptides, drugs, bio active ions, small molecules, radioactive molecules, antimicrobial molecules and radio-opaque molecules.

12. A composition according to any one of the preceding claims, further comprising a further antimicrobial substance.

13. A composition according to any one of the preceding claims, wherein the composition has a shelf-life of at least 1 years in RT.

14. An antimicrobial and/or anti-inflammatory composition according to any one of claims 1-13 for use in sterilizing a biological surface and/or a biomaterial surface *in situ.*

15. An antimicrobial and/or anti-inflammatory composition according to any one of the claims 1-14 for use in cleaning skin and/or mucosa from microbes.

16. An antimicrobial and/or anti-inflammatory composition for use according to claim 15, wherein the microbes are selected from the group consisting of bacteria and virus.

17. An antimicrobial and/or anti-inflammatory composition for use according to claim 16, wherein the microbes are Riboviria.

18. An antimicrobial and/or anti-inflammatory composition for use according to claim 16-17, wherein the microbes are selected from the group consisting of Coronaviridae.

19. An antimicrobial and/or anti-inflammatory composition for use according to claim 16-18, wherein the microbes are Orthocoronavirinae.

20. An antimicrobial and/or anti-inflammatory composition for use according to claim 16-19, wherein the microbes are Coronaviruse.

21. Application of an antimicrobial and/or anti-inflammatory composition as a hand wash, oral wash and/or for nasal and/or sinus cleansing, for washing virus off skin and mucosa.
